# EUROPEAN PATENT SPECIFICATION

(11) **EP 3 395 255 B1**
(45) Date of publication and mention of the grant of the patent: **09.12.2020**
(21) Application number: 18000320.4
(22) Date of filing: 04.04.2018
(51) Int. Cl.: A61B 6/10, A61B 6/00, H05G 1/30, H05G 1/56, G16H 40/63

(54) **X-RAY IMAGING APPARATUS**
RÖNTGENBILDGEBUNGSVORRICHTUNG
APPAREIL D'IMAGERIE PAR RAYONS X

(30) Priority: 25.04.2017 JP 2017086320
(43) Date of publication of application: 31.10.2018
(73) Proprietor: Shimadzu Corporation, Kyoto-shi Kyoto 604-8511 (JP)
(72) Inventor: Sakamoto, Yuki, Kyoto- shi Kyoto 604-8511 (JP); Okamoto, Takeshi, Kyoto- shi Kyoto 604-8511 (JP)
(74) Representative: Müller-Boré & Partner Patentanwälte PartG mbB

(56) References cited:
- JP-A- 2012 217 572
- US-A1- 2003 198 317
- US-A1- 2006 018 431

## Description

### Field

The present invention relates to an X-ray imaging apparatus obtaining X-ray images using X-rays.

### Background

The conventional X-ray imaging apparatus includes an X-ray tube for irradiating a subject with X-rays, an X-ray detector detecting X-rays having passed through the subject, an operation part receiving input operation from a user (operator), and a control unit performing control for irradiation of X-rays (see Patent Literature 1, for example).

The operation part includes an X-ray irradiation execution button (hereinafter, appropriately referred to as an "execution button") and an X-ray irradiation permission button (hereinafter, appropriately referred to as a "permission button"). The execution button is used to instruct the execution of X-ray irradiation. Meanwhile, the permission button is used to prevent X-ray irradiation not intended by a user. When the permission button is pressed to obtain an X-ray irradiation permitted state (hereinafter, appropriately referred to as a "permitted state"), a user is able to perform X-ray irradiation using the execution button. When the permission button is pressed again in such a permitted state, the state becomes an X-ray irradiation prohibited state (hereinafter, appropriately referred to as a "prohibited state"). In the prohibited state, X-ray irradiation is not possible even when a user presses the execution button.

Moreover, in the conventional X-ray imaging apparatus, the permission button includes an electric lamp. In the prohibited state, the electric lamp is lit off. Then, in the permitted state, the electric lamp is continuously lit on.

### Citation List

### Patent Literature

Patent Literature 1: Japanese Patent Application Laid-open No. 2015-009012

Document US 2006/018431 A1 may be considered to disclose a device according to the preamble of claim 1, and describes an X-ray imaging apparatus that includes a body unit including a bed unit where an object is put, an X-ray irradiation unit configured to irradiate an X-ray to the object and an X-ray detector configured to detect the X-ray passing through the object, an input unit configured to input control information to control at least part of operation of the body unit, a selection unit configured to select ON or OFF of a lock mode and a controller configured to determine at least part of the control information is locked when the lock mode is ON and the at least part of the control information is released when the lock mode is OFF.

### Summary

### Technical Problem

Each of operation buttons of the conventional X-ray imaging apparatus has a pictograph for distinction. However, even with such distinction, it may be difficult to recognize the buttons. For example, when the power source of the X-ray imaging apparatus is turned on to activate the X-ray imaging apparatus, it is in a prohibited state, and the electric lamp of the permission button is lit off. Thus, when a user operates the X-ray imaging apparatus for the first time, or he/she is not familiar with the operation, it is difficult to recognize the position of the permission button.

In view of the above-described aspect, the present invention aims at providing an X-ray imaging apparatus allowing easy recognition of the position of the permission button for X-ray irradiation in the X-ray irradiation prohibited state.

### Solution to Problem

To achieve such an object, the invention has the following configurations. That is, an X-ray imaging apparatus of the invention includes an X-ray source that irradiates a subject with X-rays, an X-ray detector that detects X-ray having passed through the subject, an operation switch group provided on an operation panel, and a permission switch for X-ray irradiation that is provided on the operation panel separately from the operation switch group and performs switching between a prohibited state prohibiting X-ray irradiation from the X-ray source and a permitted state permitting X-ray irradiation from the X-ray source, the permission switch having visual characteristics different from common visual characteristics of the operation switch group in the prohibited state.

In the X-ray imaging apparatus of the invention, the permission switch for X-ray irradiation is provided on the operation panel separately from the operation switch group, and performs switching between the prohibited state prohibiting X-ray irradiation from the X-ray source and the permitted state permitting X-ray irradiation from the X-ray source. In the prohibited state, the permission switch has visual characteristics different from common visual characteristics of the operation switch group. Thus, it is possible to emphasize the position of the permission switch to notify a user. As a result, even when a user operates the X-ray imaging apparatus for the first time, or he/she is not familiar with the operation, it is easy to recognize the position of the permission button in the X-ray irradiation prohibited state. Moreover, the position of the permission switch is emphasized, which suppresses erroneous pressing.

In the above-described X-ray imaging apparatus, it is preferable that the operation switch group is either lit on or lit off, and the permission switch blinks in the prohibited state, and is lit on instead of the blinking in the permitted state. When the permission switch blinks in the prohibited state, the position of the permission switch can be emphasized to notify a user. Moreover, the display action of the permission switch is made different between the prohibited state and the permitted state, which allows a user to recognize whether the X-ray imaging apparatus is in the prohibited state or the permitted state.

In the above-described X-ray imaging apparatus, it is preferable that the operation switch group is lit off, and the permission switch is lit on in a first color in the prohibited state, and is lit on in a second color instead of lighting in the first color in the permitted state.
When the permission switch is lit on in the first color in the prohibited state, the position of the permission switch can be emphasized to notify a user. Moreover, the display color of the permission switch is made different between the prohibited state and the permitted state, which allows a user to recognize whether the X-ray imaging apparatus is in the prohibited state or the permitted state.

In the above-described X-ray imaging apparatus, it is preferable that the permission switch includes an electric lamp unit. In the prohibited state, it is possible by the electric lamp unit to emphasize the position of the permission switch to notify a user.

The above-described X-ray imaging apparatus further includes a display unit that is provided on a back surface of a touch panel, in which a screen of the display unit preferably displays the operation switch group and the permission switch. In the prohibited state, the permission switch displayed on the screen of the display unit has visual characteristics different from common visual characteristics of the operation switch group. Thus, it is possible to emphasize the position of the permission switch to notify a user.

In the above-described X-ray imaging apparatus, it is preferable that in the prohibited state, an index figure indicating a position of the permission switch is displayed on the permission switch on the screen of the display unit or on an outer periphery of the permission switch. When the index figure indicating the position of the permission switch is displayed on the permission switch or on the outer periphery thereof in the prohibited state, the position of the permission switch can be emphasized to notify a user.

### Advantageous Effects of Invention

In the X-ray imaging apparatus of the invention, the permission switch has visual characteristics different from common visual characteristics of the operation switch group in the prohibited state. Thus, the position of the permission switch can be emphasized to notify a user. As a result, even when a user operates the X-ray imaging apparatus for the first time, or he/she is not familiar with the operation, it is easy to recognize the position of the permission button in the X-ray irradiation prohibited state.

### Brief Description of Drawings

FIG. 1 is a schematic configuration diagram of an X-ray imaging apparatus according to a first example.
FIG. 2 is a diagram illustrating an example of an operation panel according to the first example.
FIG. 3 (a), (b) are diagrams for explaining the arrangement of an electric lamp unit.
FIG. 4 is a diagram for explaining the operation of an electric lamp unit of an X-ray irradiation permission button according to the first example.
FIG. 5 is a diagram for explaining the operation of an electric lamp unit of an X-ray irradiation permission button according to a second example.
FIG. 6 is a diagram illustrating an example of an operation panel according to a third example.
FIG. 7 is a diagram for explaining blinking action according to the third example.
FIG. 8 (a) to (e) are diagrams illustrating examples of an index figure or an index character according to a fourth example.

### Description of Embodiments

### [First example]

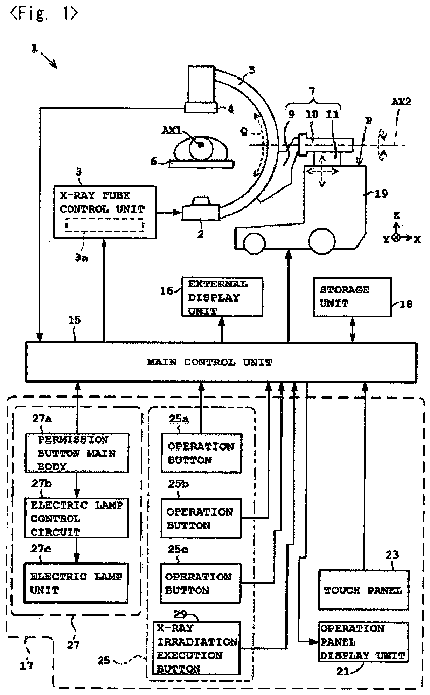

The following will describe the first example of the invention with reference to the enclosed drawings. FIG. 1 is a schematic configuration diagram of an X-ray imaging apparatus according to the first example.

### <Configuration of X-ray imaging apparatus 1>

FIG. 1 is referred to. An X-ray imaging apparatus 1 includes an X-ray tube 2, an X-ray tube control unit 3, an X-ray detector 4, a C arm 5, a top board 6, and an arm rotary moving mechanism 7. The X-ray tube 2 irradiates a subject M placed on the top board 6 with X-rays. The X-ray tube 2 is controlled by the X-ray tube control unit 3. The X-ray tube 2 corresponds to an X-ray source of the invention. The X-ray tube control unit 3 includes an X-ray control circuit (not illustrated) and a high voltage generation part 3a, and supplies a predetermined tube voltage and tube current to the X-ray tube 2.

The X-ray detector 4 is arranged to face the X-ray tube 2, and detects X-rays having passed through the subject M. The X-ray detector 4 is formed by a flat panel type X-ray detector (FPD), or an image intensifier and a camera, for example. The images captured by the X-ray detector 4 are subjected to necessary image processing such as various kinds of correction by an image processing unit (not illustrated).

The X-ray tube 2 and the X-ray detector 4 are arranged to face each other and supported by the C arm 5. The C arm 5 is formed to be moved and rotated by the arm rotary moving mechanism 7. The arm rotary moving mechanism 7 includes a sliding mechanism 9, a rotation mechanism 10, and an upper/lower/front/rear moving mechanism 11.

The sliding mechanism 9 slides the C arm 5 along a C shape of the C arm 5, as illustrated by an arrow Q. Thus, the X-ray tube 2 and the X-ray detector 4 rotate around a first axis AX1. The rotation mechanism 10 rotates the sliding mechanism 9 around a second axis AX2. In this manner, the X-ray tube 2 and the X-ray detector 4 rotate around the second axis AX2. The upper/lower/front/rear moving mechanism 11 moves the rotation mechanism 10 in an upper/lower direction (Z direction) and a front/rear direction (X direction). Thus, the X-ray tube 2 and the X-ray detector 4 move in the upper/lower direction (Z direction) and the front/rear direction (X direction). Each of the sliding mechanism 9, the rotation mechanism 10, and the upper/lower/front/rear moving mechanism 11 has an electric motor.

The X-ray imaging apparatus 1 further includes a main control unit 15, an external display unit 16, an operation panel 17, and a storage unit 18. The main control unit 15 includes a central processing unit (CPU). The main control unit 15 controls the components such as the X-ray tube control unit 3, the X-ray detector 4, the arm rotary moving mechanism 7 (sliding mechanism 9, rotation mechanism 10, and upper/lower/front/rear moving mechanism 11), and an operation panel display unit 21 described later. The external display unit 16 is formed by a liquid crystal display (LCD), and the like. The configuration of the operation panel 17 will be described later. The storage unit 18 is formed by a read-only memory (ROM), a random-access memory (RAM), or a hard disc, and the like. The storage unit 18 stores programs necessary for the operation of the X-ray imaging apparatus 1.

The X-ray imaging apparatus 1 is mobile and includes a carriage 19. The carriage 19 supports the upper/lower/front/rear moving mechanism 11, and supports the X-ray tube 2 and the X-ray detector 4 through the upper/lower/front/rear moving mechanism 11 and the like. Moreover, there are mounted, on the carriage 19, the X-ray tube control unit 3, the main control unit 15, the operation panel 17, the storage unit 18, and the like. The operation panel 17 is provided on a surface of the carriage 19, which is a position illustrated by an arrow P, for example. Note that although the X-ray imaging apparatus 1 is mobile in the example, it may not be mobile.

### [Configuration of operation panel 17]

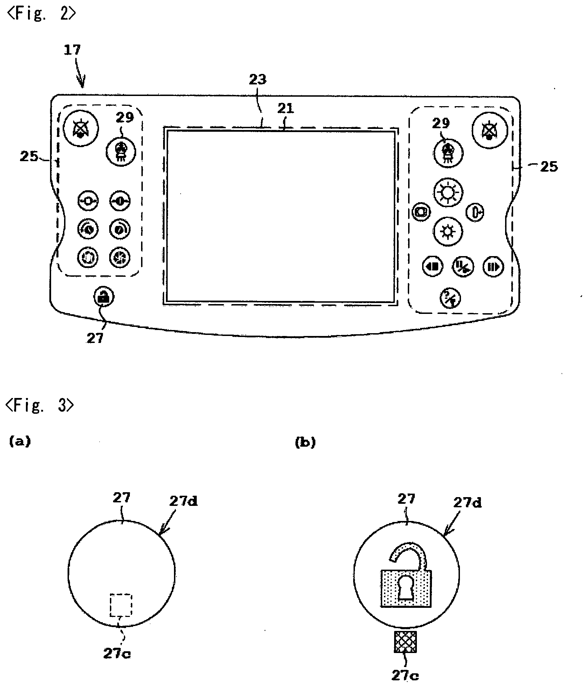

The following will describe the configuration of the operation panel 17. FIG. 1 illustrates a block diagram of the operation panel 17. FIG. 2 is a diagram illustrating an example of the operation panel 17. The operation panel 17 includes an operation panel display unit 21, a touch panel 23, an operation button group 25, and an X-ray irradiation permission button 27. In FIG. 1, three operation buttons 25a to 25c are illustrated as the operation button group 25. However, the number of operation buttons is not limited to three. The operation button group 25 corresponds to an operation switch group of the invention, and the X-ray irradiation permission button 27 corresponds to a permission switch of the invention.

The operation panel display unit 21 is formed by a liquid crystal display or an organic EL display, for example. The touch panel 23 is provided to correspond to the operation panel display unit 21, and is provided on a front surface of the operation panel display unit 21. The touch panel 23 is formed with a detection type such as a resistive film type. The operation button group 25 provided on the operation panel 17 is provided with a plurality of operation buttons including an X-ray irradiation execution button 29. The operation buttons of the operation button group 25 are formed with physical switches (mechanical switches) called tactile switches or push switches, for example. The execution button 29 is used to instruct the execution of X-ray irradiation from the X-ray tube 2. In FIG. 2, two execution buttons 29 are provided, and X-ray irradiation is performed by pressing any one of them.

The permission button 27 is provided on the operation panel 17 separately from the operation button group 25. The permission button 27 is used to perform switching between the X-ray irradiation prohibited state in which X-ray irradiation from the X-ray tube 2 is prohibited and the X-ray irradiation permitted state in which X-ray irradiation from the X-ray tube 2 is permitted. The permission button 27 includes a permission button main body 27a, an electric lamp control circuit 27b, and an electric lamp unit 27c, as illustrated in FIG. 1. The permission button main body 27a is formed by physical switches (mechanical switches) called tactile switches or push switches, for example. The electric lamp control circuit 27b controls the electric lamp unit 27c based on operation information of the permission button main body 27a. The electric lamp control circuit 27b is formed with or without a CPU. The electric lamp unit 27c includes one electric lamp. As the electric lamp, there is used a light emitting diode (LED), a bulb, or EL lightning, for example. The operation button group 25 does not include an electric lamp unit such as the permission button 27.

FIG. 3(a) and FIG. 3(b) are diagrams for explaining the arrangement of the electric lamp unit 27c. In FIG. 3(a), a pictograph of a lock is omitted. The electric lamp unit 27c is embedded to emit light inside a circular area 27d for pressing the permission button 27, as illustrated in FIG. 3(a). Moreover, the electric lamp unit 27c may be provided to emit light outside (on an outer periphery of) the circular area 27d, as illustrated in FIG. 3(b).

In the prohibited state, the permission button 27 has visual characteristics different from common visual characteristics of the operation button group 25. In the example, the permission button 27 blinks by the electric lamp unit 27c in the prohibited state. Moreover, the permission button 27 is continuously lit on instead of blinking by the electric lamp unit 27c in the permitted state. Here, the operation button group 25 does not include an electric lamp unit, and thus it is regarded that the operation button group 25 is lit off. Note that the common visual characteristics of the operation button group 25 indicate that the operation buttons have an almost same color or printing of characters or pictographs in a same color, or it is not blinking (either lit on or off), for example. When the operation button group 25 is lit on or off, each button (switch) of the operation button group 25 is provided with an electric lamp unit.

### <Operation of X-ray imaging apparatus 1>

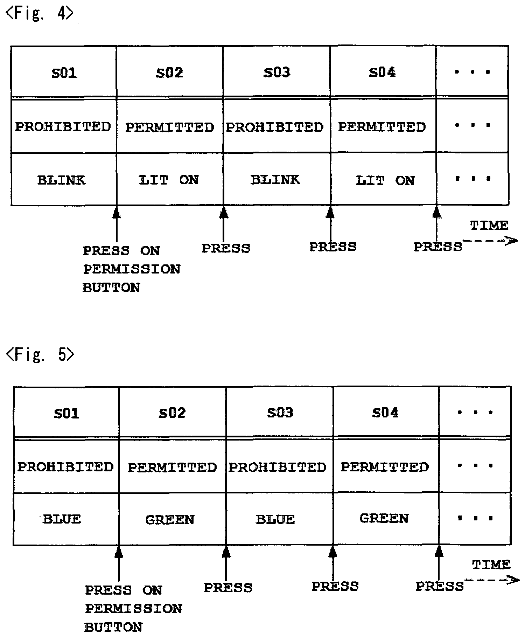

The following will describe the operation of the X-ray imaging apparatus 1. FIG. 4 is a diagram for explaining the operation of the electric lamp unit 27c of the permission button 27.

In the X-ray imaging apparatus 1, for example, the apparatus 1 is activated when the power source is turned on, and it is in the prohibited state in which X-ray irradiation from the X-ray tube 2 is prohibited. Conventionally, the electric lamp unit 27c of the permission button 27 is lit off in the prohibited state. Meanwhile, each of the operation buttons has a pictograph for distinction. However, even with such distinction, when a user operates the X-ray imaging apparatus 1 for the first time, or he/she is not familiar with the operation, it may be difficult to recognize the position of the permission button 27. Thus, the permission button 27 blinks in the prohibited state. Moreover, the permission button 27 is lit on instead of blinking in the permitted state.

The description will be given concretely. The X-ray irradiation permission button 27 includes the electric lamp unit 27c. The permission button 27 blinks, as visual characteristics, by the electric lamp unit 27c in the prohibited state (see Step S01 of FIG. 4). The blinking control is performed by the electric lamp control circuit 27b. The electric lamp control circuit 27b controls one electric lamp of the electric lamp unit 27c to blink. Thus, the permission button 27 blinks. When the X-ray imaging apparatus 1 is in the prohibited state, X-ray tube 2 does not emit X-rays even when the X-ray irradiation execution button 29 is pressed. Note that the blinking of the example indicates that lighting on and off are performed alternately, and is not for adjusting the luminance of the electric lamp continuously lit on by changing a blinking speed.

The permission button 27 is operated to perform switching between the prohibited state and the permitted state. When the blinking permission button 27 is pressed in the prohibited state, the prohibited state is switched to the permitted state. In the permitted state, the permission button 27 is continuously lit on instead of blinking of the permission button 27 (see Step S02 of FIG. 4). The electric lamp control circuit 27b lights one electric lamp of the electric lamp unit 27c based on operation information (signals) indicating that the permission button 27 has been pressed. In this manner, the permission button 27 is lit on. The permission button 27 blinks in the prohibited state, while it is lit on in the permitted state, which makes it possible to recognize whether the X-ray imaging apparatus 1 is in the prohibited state or the permitted state.

The operation information indicating that the permission button 27 has been pressed is transmitted not only to the electric lamp control circuit 27b but also to the main control unit 15. Based on such operation information, the main control unit 15 changes the state of the X-ray imaging apparatus 1 from the prohibited state to the permitted state, and it changes the state from the permitted state to the prohibited state.

The following will describe the operation of obtaining an X-ray image. The subject M is placed on the top board 6 illustrated in FIG. 1. The permission button 27 is lit on, whereby a user recognizes the permitted state. In the permitted state, the X-ray tube 2 emits X-rays when the execution button 29 is pressed. That is, when the execution button 29 is pressed, the operation information indicating that the execution button 29 has been pressed is transmitted to the X-ray tube control unit 3 through the main control unit 15. The X-ray tube control unit 3 supplies a necessary tube voltage and tube current to the X-ray tube 2, so that the X-ray tube 2 emits X-rays. The X-rays that have been emitted by the X-ray tube 2 and have passed through the subject M is detected by the X-ray detector 4. The X-ray detector 4 outputs an X-ray image in accordance with the detected X-ray amount. The output X-ray image is subjected to necessary processing such as gradation conversion by an image processing unit (not illustrated) or the main control unit 15. Then, the X-ray image is displayed on the external display unit 16 and the operation panel display unit 21, and stored in the storage unit 18.

When the lit permission button 27 is pressed in the permitted state, the permitted state is switched to the prohibited state. In the prohibited state, the permission button 27 blinks (see Step S03 of FIG. 4). Then, every time the permission button 27 is pressed, the prohibited state and the permitted state are switched alternately (see Steps S01 to S04 of FIG. 4).

In the example, the operation button group 25 does not include an electric lamp unit and is not lit on. Meanwhile, the permission button 27 includes the electric lamp unit 27c and blinks. The blinking has a higher emphasis level than pictographs on the operation buttons of the operation button group 25. Thus, a user can easily recognize the position of the permission button 27 even when the permission button 27 is arranged in the operation button group 25.

In the example, the permission button 27 for X-ray irradiation is provided on the operation panel 17 separately from the operation button group 25, and performs switching between the prohibited state in which X-ray irradiation from the X-ray tube 2 is prohibited and the permitted state in which X-ray irradiation from the X-ray tube 2 is permitted. Then, the permission button 27 has visual characteristics different from common visual characteristics of the operation button group 25 in the prohibited state. In the example, the permission button 27 includes the electric lamp unit 27c, and blinks by the electric lamp unit 27c in the prohibited state. In this manner, the position of the permission button 27 can be emphasized to notify a user. As a result, even when a user operates the X-ray imaging apparatus 1 for the first time, or he/she is not familiar with the operation, it is easy to recognize the position of the permission button 27 in the prohibited state.

Moreover, when the permission button 27 is provided at a position easy to press, it is possible to cause error operation such as erroneous emission of X-rays. Thus, the permission button 27 is arranged at a position relatively hard to press, which is, for example, a position where other operation button group 25 is arranged. When the permission button 27 is arranged at such a position, it cannot be found at a glance, increasing the trouble for looking for the permission button 27. Moreover, when other operation button group 25 is concentrated or when the operation panel display unit 21 exists, it is necessary to find out the permission button 27 among them, which is more troublesome. Even in such cases, the example facilitates recognition of the position of the permission button 27 in the prohibited state.

Moreover, the permission button 27 blinks by the electric lamp unit 27c in the prohibited state, and is continuously lit on instead of blinking by the electric lamp unit 27c in the permitted state. The light emitting action of the permission button 27 is made different between the prohibited state and the permitted state, which allows a user to recognize whether the X-ray imaging apparatus 1 is in the prohibited state or the permitted state. Even when the permission button 27 is pressed wrongly, a user can recognize whether the X-ray imaging apparatus 1 is in the prohibited state or the permitted state.

Moreover, the blinking has a high emphasis level. If non-blinking operation buttons are provided on the screen of the operation panel display unit 21, it is possible to distinguish the permission button 27 not only from the physical operation button group 25 outside the operation panel display unit 21 but also from such operation buttons displayed on the screen.

### [Second example]

The following will describe the second example of the invention with reference to the enclosed drawings. The description overlapping the first example will be omitted. FIG. 5 is a diagram for explaining the operation of the electric lamp unit 27c of the permission button 27.

In the first example, the permission button 27 blinks in the prohibited state, while is continuously lit on in the permitted state, by the electric lamp unit 27c. That is, the light emitting action is changed depending on whether the X-ray imaging apparatus 1 is in the prohibited state or the permitted state. In this aspect, a light emission color is changed in the second example.

In the example, the electric lamp unit 27c illustrated in FIG. 1 includes a plurality of (two or more) electric lamps emitting light mutually different in color. For example, the electric lamp unit 27c includes a first electric lamp emitting blue light and a second electric lamp emitting green light. Note that the light emission color is not limited to blue and green.

As illustrated in FIG. 5, the permission button 27 is lit on in blue as a first color in the prohibited state, while it is lit on in green as a second color instead of lighting in blue, by the electric lamp unit 27c. The electric lamp control circuit 27b controls the first electric lamp of the electric lamp unit 27c to emit blue light, and controls the second electric lamp of the electric lamp unit 27c to emit green light. Note that blue light and green light may be emitted reversely. That is, the permission button 27 is lit on in green in the prohibited state, and is lit on in blue instead of green in the permitted state. The first color or the second color may be a light blue, for example, combining the light emitted by the first electric lamp and the second electric lamp. The operation button group 25 is not provided with an electric lamp unit, and thus regarded that it is lit off.

In the example, the permission button 27 is lit on in blue as the first color by the electric lamp unit 27c in the prohibited state. When the permission button 27 is lit on in the prohibited state, the position of the permission button 27 can be emphasized to notify a user. Moreover, the permission button 27 is lit on in green as the second color instead of blue by the electric lamp unit 27c in the permitted state. The light emission color of the permission button 27 is made different between the prohibited state and the permitted state, which allows a user to recognize whether the X-ray imaging apparatus 1 is in the prohibited state or the permitted state.

The first example and the second example may be combined. In this case, the permission button 27 blinks in blue in the prohibited state, and is lit on in green instead of blinking in blue in the permitted state.

### [Third example]

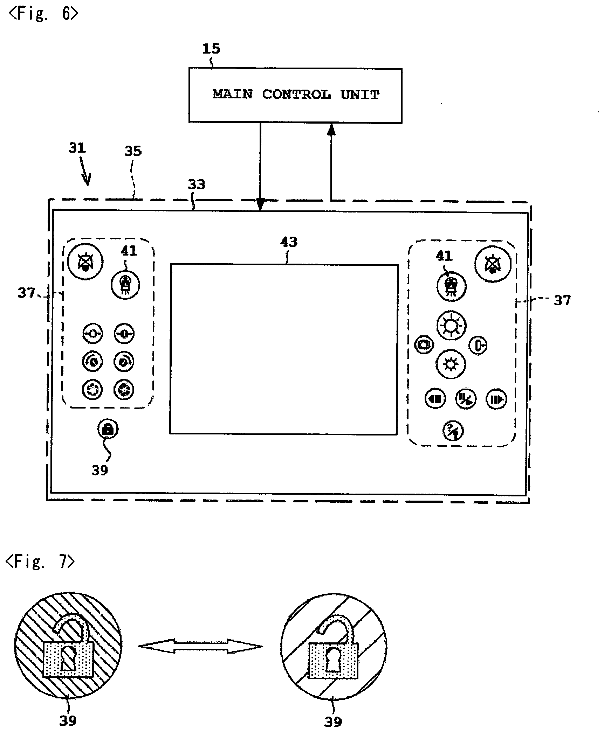

The following will describe the third example of the invention with reference to the enclosed drawings. The description overlapping the first example and the second example will be omitted. FIG. 6 is a diagram illustrating an example of an operation panel 31. FIG. 7 is a diagram for explaining blinking action.

In the first example, the permission button 27 blinks by the electric lamp unit 27c in the prohibited state. Regarding this aspect, in the third example, a permission button 39 displayed on a screen of a display unit provided on a back surface of a touch panel blinks in the prohibited state. That is, in the third example, the permission button 39 displayed on the screen of the display unit, not a physical button, blinks.

FIG. 6 is referred to. The operation panel 31 includes an operation panel display unit 33 and a touch panel 35. The operation panel display unit 33 is formed by a liquid crystal display or an organic EL display, for example. The operation panel display unit 33 corresponds to a display unit of the invention. The touch panel 35 is provided on a front surface of the operation panel display unit 33. On the screen of the operation panel display unit 33 provided on a back surface of the touch panel 35, there are displayed an operation button group 37 and a permission button 39. When a user presses (touches) buttons such as the permission button 39 displayed on the screen through the touch panel 35, the input of the buttons is performed. Note that a symbol 41 indicates an X-ray irradiation execution button. An area 43 indicates an area where X-ray conditions or X-ray images are displayed.

Similarly to the first example, the permission button 39 displayed on the screen blinks in the prohibited state. The description will be given concretely. For example, it is assumed that the X-ray imaging apparatus 1 is in the prohibited state. In this case, the main control unit 15 displays the permission button 39 on the screen of the operation panel display unit 33 alternately in a color (e.g., blue) different from the common color (e.g., grey) of the operation button group 37 and in a color (e.g., grey) other than such a color. That is, as illustrated in FIG. 7, the permission buttons 39 with a mutually different back color of a pictograph of a lock on the right and left sides are displayed alternately. Thus, the permission button 39 blinks for display.

Once the blinking permission button 39 is pressed in the prohibited state, the prohibited state is switched to the permitted state. The main control unit 15 controls the permission button 39 on the screen to be displayed in the above-described different color (e.g., blue) without blinking in the permitted state. When the non-blinking permission button 39 is pressed in the permitted state, the permitted state is switched to the prohibited state.

In the example, the permission button 39 displayed on the screen blinks in the prohibited state. When the permission button 39 blinks in the prohibited state, the position of the permission button 39 can be emphasized to notify a user. Moreover, the permission button 39 blinks, in the prohibited state, by displaying it alternately in a color (e.g., blue) different from the common color (e.g., grey) of the operation button group 37 and in a color (e.g., grey) other than such a color. By contrast, the permission button 39 is displayed, in the permitted state, in a different color (e.g., blue) different from the above-described common color instead of blinking. The display method of the permission switch is made different between the prohibited state and the permitted state, which allows a user to recognize whether the X-ray imaging apparatus 1 is in the prohibited state or the permitted state.

Moreover, the blinking has a high emphasis level. If non-blinking operation buttons are provided outside the operation panel display unit 33 in the operation panel 31, it is possible to distinguish the permission button 39 not only from the operation button group 37 displayed on the screen of the operation panel display unit 33 but also from such operation buttons displayed outside the operation panel display unit 33.

Note that a modification of the example will be described. First, the modification may be configured in the same manner as in the second example. That is, the permission button 39 may be displayed in a first color (e.g., blue) different from the common color in the prohibited state, while it may be displayed in a second color (e.g., green) different from the common color in the permitted state (both cases without blinking). Moreover, the modification may be configured in the same manner as the combination of the first example and the second example. That is, the permission button 39 may blink by displaying it alternately in a first color (e.g., blue) different from the common color and a color other than such a color in the prohibited state, while it may be displayed in a second color (e.g., green) different from the common color in the permitted state.

### [Fourth example]

The following will describe the fourth example of the invention with reference to the enclosed drawings. The description overlapping the first example to the third example will be omitted.

In the third example, the display action such as blinking of the permission button 39 displayed on the screen is changed. In the fourth example, the following configuration is possible in addition to the third example or individually. In the prohibited state, there is displayed, on the permission button 39 or on the outer periphery thereof, at least one of an index figure and an index character indicating a position of the permission button 39 as a guide for a user. The index figure and the index character are displayed on the screen of the operation panel display unit 33 provided on the back surface of the touch panel 35.

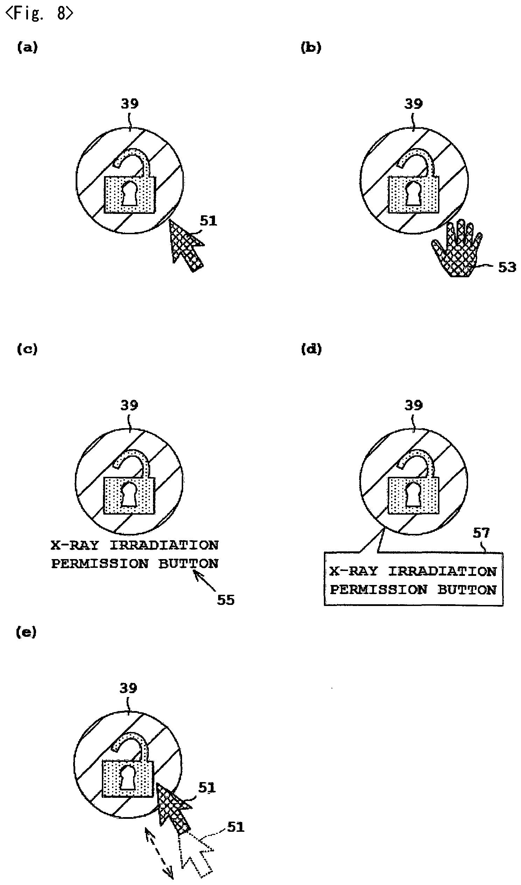

FIG. 8(a) to FIG. 8(e) are diagrams illustrating examples of an index figure or an index character. In FIG. 8(a), an arrow shape 51 is displayed as an index figure on the outer periphery of the permission button 39. In FIG. 8(b), a hand shape 53 is displayed as an index figure. In FIG. 8(c), a character 55 such as "X-ray irradiation permission button" is displayed as an index character on the outer periphery of the permission button 39. In FIG. 8(d), a character such as "X-ray irradiation permission button" is displayed in a balloon 57 that is a frame for writing in characters, as the combination of an index figure and an index character. In FIG. 8(e), the arrow shape 51 is not still but moving for display. Moreover, the index figure and the index character may blink for display in addition to the moving display or individually.

In the example, the index figure or the index character indicating a position of the permission button 39 is displayed, in the prohibited state, on the permission button 39 on the screen of the operation panel display unit 33 or on the outer periphery thereof. When the index figure or the index character indicating a position of the permission button 39 is displayed on the permission button 39 or on the outer periphery thereof in the prohibited state, the position of the permission button 39 can be emphasized to notify a user.

Note that in the permitted state, the index figure or the index character may not be displayed. Alternatively, the index figure or the like may be changed in color. Moreover, as long as the index figure or the like is moving in the prohibited state, the index figure or the like may be still in the permitted state. Moreover, as long as the index figure or the like blinks in the prohibited state, the index figure or the like may not blink in the permitted state.

The invention is not limited to the above-described embodiments, and can be modified and implemented in the following manners.
(1) In the above-described examples 1, 2, the electric lamp unit 27c includes one electric lamp. However, the number of electric lamps is not limited to one. That is, the electric lamp unit 27c may include at least one electric lamp. For example, it is assumed that the electric lamp unit 27c includes two electric lamps. In the prohibited state, the first electric lamp may blink, while the second electric lamp may be lit off. In the permitted state, the first electric lamp may be lit off, while the second electric lamp may be lit on. Moreover, both of the two electric lamps may be synchronized to blink in the prohibited state, while both of the two electric lamps are lit on in the permitted state, for example.
(2) In the above-described first and second examples and the modification (1), the permission button 27 blinks in the prohibited state, and is lit on in the permitted state. In this aspect, the action of blinking and lighting may be opposite. That is, the permission button 27 is lit on in the prohibited state, and blinks instead of lighting in the permitted state. When the permission button 27 is lit on in the prohibited state, the position of the permission button 27 can be emphasized to notify a user. The light emitting action of the permission switch is made different between the prohibited state and the permitted state, which allows a user to recognize whether the X-ray imaging apparatus 1 is in the prohibited state or the permitted state.
(3) In the above-described first and second examples and modification (1), the permission button 27 blinks in the prohibited state, and is lit on in the permitted state. For example, the permission button 27 may act by either blinking or being lit on in the both prohibited state and permitted state. This exerts the effect of facilitating the recognition of a position of the permission button 27 in the prohibited state. However, a user cannot recognize whether the X-ray imaging apparatus 1 is in the prohibited state or in the permitted state.
(4) In the above-described first example, the permission button 27 blinks by the electric lamp unit 27c, for example, as visual characteristics different from common visual characteristics of the operation button group 25. In this aspect, the permission button 27 may be colored with blue paint, for example, as visual characteristics different from common visual characteristics (e.g., white) of the operation button group 25.
(5) In the above-described first and second examples and modifications, the permission button 27 blinks, for example, in the prohibited state. This also exerts the effect of suppressing erroneous pressing (misoperation) of pressing the permission button 27 wrongly. To suppress such erroneous pressing on the permission button 27, the permission button 27 may be provided with a transparent cover member opened and closed like a door in the front surface of the permission button 27. Moreover, with a plurality of (e.g., two) permission buttons 27, the prohibited state and the permitted state may be switched when such permission buttons 27 are pressed at the same time. Alternatively, the prohibited state and the permitted state may be switched by pressing the permission button 27 twice such as a double click or by pressing it for a long time.
(6) In the above-described examples and modifications, the permission button 27, 39 is lit on in the permitted state. In this aspect, the permission button 27, 39 may be lit off in the permitted state. Even in this case, the permission button 27, 39 blinks in the prohibited state. Thus, in the prohibited state, the position of the permission button 27, 39 can be emphasized to notify a user. Note that in the third example, being lit off indicates that the permission button is displayed in the common color (e.g., grey) of the operation button group 37, and being lit on indicates that the permission button is displayed in a color (e.g., blue or green) different from the common color (e.g., grey).
(7) In the above-described examples and modifications, the operation button group 25, 37, and the permission button 27, 39 are buttons (button switches). However, they may be other kinds of switches such as sliding type switches or toggle switches.

### Reference Signs List

1 X-ray imaging apparatus
2 X-ray tube
4 X-ray detector
5 main control unit
17, 31 operation panel
21, 33 operation panel display unit
23, 35 touch panel
25, 37 operation button group
27, 39 X-ray irradiation permission button (permission button)
27a permission button main body
27b electric lamp control circuit
27c electric lamp unit
29, 41 X-ray irradiation execution button (execution button)

## Claims

1. An X-ray imaging apparatus (1), comprising:
an X-ray source (2) that irradiates a subject with X-rays;
an X-ray detector (4) that detects X-rays having passed through the subject;
an operation switch group (25) provided on an operation panel (17, 31); and
a permission switch (27, 39) for X-ray irradiation that is provided on the operation panel (17, 31) separately from the operation switch group (25) and performs switching between a prohibited state prohibiting X-ray irradiation from the X-ray source (2) and a permitted state permitting X-ray irradiation from the X-ray source (2), **characterised by** the permission
switch (27, 39) being lit on in the permitted state, and the permission switch (27, 39), in the prohibited state, changing a state, wherein in said prohibited state the permission switch (27, 39) is lit on to be different from the operation switch group (25) and the permission switch (27, 39) in the permitted state.

2. The X-ray imaging apparatus (1) according to claim 1, wherein the operation switch group (25) is either lit on or lit off, and the permission switch (27, 39) blinks in the prohibited state and is lit on instead of the blinking in the permitted state.

3. The X-ray imaging apparatus (1) according to claim 1, wherein the operation switch group (25) is lit off, and the permission switch (27, 39) is lit on in a first color in the prohibited state and is lit on in a second color instead of lighting in the first color in the permitted state.

4. The X-ray imaging apparatus (1) according to any one of claims 1 to 3, wherein the permission switch (27, 39) includes an electric lamp unit (27c).

5. The X-ray imaging apparatus (1) according to any one of claims 1 to 3, further comprising a display unit (16) that is provided on a back surface of a touch panel (23, 35), wherein a screen of the display unit (16) displays the operation switch group (25) and the permission switch (27, 39).

6. The X-ray imaging apparatus (1) according to claim 5, wherein in the prohibited state, an index figure indicating a position of the permission switch (27, 39) is displayed on the permission switch (25) on the screen of the display unit (16) or on an outer periphery of the permission switch (25).

## Patentansprüche

1. Eine Röntgenbildgebungsvorrichtung (1), die Folgendes umfasst:
eine Röntgenquelle (2), die ein Subjekt mit Röntgenstrahlen bestrahlt;
einen Röntgendetektor (4), der Röntgenstrahlen detektiert, die durch das Subjekt hindurchgegangen sind;
eine Betätigungs-Schaltergruppe (25), die auf einer Betätigungs-Konsole (17, 31) vorgesehen ist; und
einen Erlaubnis-Schalter (27, 39) zur Röntgenbestrahlung, der auf der Betätigungs-Konsole (17, 31) getrennt von der Betätigungs-Schaltergruppe (25) vorgesehen ist und das Umschalten durchführt zwischen einem verbotenen Zustand, der eine Röntgenbestrahlung aus der Röntgenquelle (2) verbietet, und einem erlaubten Zustand, der eine Röntgenbestrahlung aus der Röntgenquelle (2) erlaubt,
**dadurch gekennzeichnet, dass** der Erlaubnis-Schalter (27, 39) im erlaubten Zustand durchgehend beleuchtet ist bzw. leuchtet (*being lit on*) und der Erlaubnis-Schalter (27, 39) im verbotenen Zustand einen Zustand ändert, wobei im genannten verbotenen Zustand der Erlaubnis-Schalter (27, 39) so leuchtet, dass er sich von der Betätigungs-Schaltergruppe (25) und dem Erlaubnis-Schalter (27, 39) im erlaubten Zustand unterscheidet.

2. Röntgenbildgebungsvorrichtung (1) nach Anspruch 1, wobei die Betätigungs-Schaltergruppe (25) entweder leuchtet oder nicht leuchtet und der Erlaubnis-Schalter (27, 39) im verbotenen Zustand blinkt und im erlaubten Zustand anstelle des Blinkens durchgehend leuchtet.

3. Die Röntgenbildgebungsvorrichtung (1) nach Anspruch 1, wobei die Betätigungs-Schaltergruppe (25) ausgeschaltet ist und der Erlaubnis-Schalter (27, 39) im verbotenen Zustand in einer ersten Farbe durchgehend leuchtet und im erlaubten Zustand in einer zweiten Farbe durchgehend leuchtet, anstatt in der ersten Farbe durchgehend zu leuchten.

4. Die Röntgenbildgebungsvorrichtung (1) nach einem von Anspruch 1 bis 3, wobei der Erlaubnis-Schalter (27, 39) eine elektrische Lampeneinheit (27c) beinhaltet.

5. Die Röntgenbildgebungsvorrichtung (1) nach irgendeinem der Ansprüche von 1 bis 3, die ferner eine Anzeigeeinheit (16) umfasst, die an einer Rückseite eines Touchpanels (23, 35) bereitgestellt wird, wobei ein Bildschirm der Anzeigeeinheit (16) die Betätigungs-Schaltergruppe (25) und den Erlaubnis-Schalter (27, 39) anzeigt.

6. Die Röntgenbildgebungsvorrichtung (1) nach Anspruch 5, wobei im verbotenen Zustand eine Indexzahl, die eine Position des Erlaubnis-Schalters (27, 39) angibt, auf dem Erlaubnis-Schalter (25) auf dem Bildschirm der Anzeigeeinheit (16) oder auf einem äußeren Umfang des Erlaubnis-Schalters (25) angezeigt wird.

## Revendications

1. Un appareil d'imagerie par rayons X (1), comprenant :
une source de rayons X (2) qui irradie un sujet avec des rayons X ;
un détecteur de rayons X (4) qui détecte les rayons X ayant traversé le sujet ;
un groupe de commutateurs d'actionnement (25) prévu sur un panneau de commande (17, 31) ; et
un commutateur d'autorisation (27, 39) pour l'irradiation par rayons X qui est fourni sur le panneau de commande (17, 31) séparément du groupe de commutateurs d'actionnement (25) et qui effectue la commutation entre un état interdit interdisant l'irradiation par rayons X en provenance de la source de rayons X (2) et un état autorisé permettant l'irradiation par rayons X en provenance de la source de rayons X (2),
**caractérisé en ce que** le commutateur d'autorisation (27, 39) est allumé dans l'état autorisé, et que le commutateur d'autorisation (27, 39), dans l'état interdit, change un état, sachant que dans ledit état interdit, le commutateur d'autorisation (27, 39) est allumé pour être différent du groupe de commutateurs d'actionnement (25) et du commutateur d'autorisation (27, 39) dans l'état autorisé.

2. L'appareil d'imagerie par rayons X (1) d'après la revendication 1, sachant que le groupe de commutateurs d'actionnement (25) est soit allumé soit éteint, et que le commutateur d'autorisation (27, 39) clignote dans l'état interdit et est allumé au lieu de clignoter dans l'état autorisé.

3. L'appareil d'imagerie par rayons X (1) d'après la revendication 1, sachant que le groupe de commutateurs d'actionnement (25) est éteint et que le commutateur d'autorisation (27, 39) est allumé dans une première couleur à l'état interdit et est allumé dans une deuxième couleur au lieu de s'allumer dans la première couleur à l'état autorisé.

4. L'appareil d'imagerie par rayons X (1) d'après l'une quelconque des revendications de 1 à 3, sachant que le commutateur d'autorisation (27, 39) inclut une unité lampe électrique (27c).

5. L'appareil d'imagerie par rayons X (1) d'après l'une quelconque des revendications de 1 à 3, comprenant en outre une unité d'affichage (16) qui est prévue sur une surface arrière d'un panneau tactile (23, 35), sachant que un écran de l'unité d'affichage (16) affiche le groupe de commutateurs d'actionnement (25) et le commutateur d'autorisation (27, 39).

6. L'appareil d'imagerie par rayons X (1) d'après la revendication 5, sachant que dans l'état interdit, un chiffre d'index indiquant une position du commutateur d'autorisation (27, 39) est affiché sur le commutateur d'autorisation (25) sur l'écran de l'unité d'affichage (16) ou sur une périphérie extérieure du commutateur d'autorisation (25).
